(19) Europäisches Patentamt
European Patent Office
Office européen des brevets

(11) **EP 1 305 599 B1**

(12) **EUROPEAN PATENT SPECIFICATION**

(45) Date of publication and mention
of the grant of the patent:
**02.06.2010 Bulletin 2010/22**

(51) Int Cl.:
***G01N 21/27*** *(2006.01)*    ***G01N 21/33*** *(2006.01)*
***A61L 2/20*** *(2006.01)*

(21) Application number: **01924058.9**

(22) Date of filing: **20.04.2001**

(86) International application number:
**PCT/SE2001/000865**

(87) International publication number:
**WO 2001/092854 (06.12.2001 Gazette 2001/49)**

(54) **A METHOD AND AN APPARATUS FOR PRODUCING A GASEOUS MEDIUM**

VERFAHREN UND VORRICHTUNG ZUR HERSTELLUNG EINES GASFÖRMIGEN MEDIUMS

PROCEDE ET APPAREIL PERMETTANT DE PRODUIRE UNE SUBSTANCE GAZEUSE

(84) Designated Contracting States:
**AT BE CH CY DE DK ES FI FR GB GR IE IT LI LU
MC NL PT SE TR**

(30) Priority: **31.05.2000 SE 0002040**

(43) Date of publication of application:
**02.05.2003 Bulletin 2003/18**

(73) Proprietor: **Tetra Laval Holdings & Finance SA
1009 Pully (CH)**

(72) Inventor: **HALLSTADIUS, Hans
S-226 70 Lund (SE)**

(74) Representative: **Persson, Eva Kerstin Ch. et al
AB Tetra Pak,
Patent Department,
Ruben Rausings Gata
221 86 Lund (SE)**

(56) References cited:
**EP-A2- 0 916 937       US-A- 4 296 068
US-A- 5 847 393       US-A- 5 866 356
US-A- 5 872 359       US-A- 6 063 631**

• **TAIZO I, SINICHI A, KAWAMURA K: "Application
of a newly developed hydrogen peroxide vapor
phase sensor to HPV steriliser" PDA JOURNAL
OF PHARMACEUTICAL SCIENCE &
TECHNOLOGY, vol. 52, no. 1, 1 January 1998
(1998-01-01), pages 13-17, XP001083519**

Description

## TECHNICAL FIELD

The present invention relates to a method for producing a gaseous medium containing a sterilising agent, as well as to regulating and monitoring the concentration and quality of the gaseous sterilisation medium.

The present invention also relates to a system for producing a gaseous medium containing a sterilisation agent, as well as for continously regulating and monitoring the concentration and quality of the gaseous sterilisation medium.

The present invention particularly relates to reducing the method and the system into practice in applications for sterilising packing and filling machines, packages or packaging material employing a gaseous sterilisation medium.

## BACKGROUND ART

In processes for packing food products, it is crucial to keep the level of bacteria and other micro-organisms low in order to be able to realise food products of high quality and long shelf life which permit long transport haulage distances and distribution over long distances while the food product may still be kept fresh and unaffected by bacteria. Depending upon the type of food product which is to be packed, the sterilisation step is more or less critical. In particular with respect to aseptic dairy products, i.e. dairy products for lengthy storage at room temperature, it is most vital that the food product as well as the package be completely sterilised before filling and sealing of the package and that the risk that the food and packaging material be recontaminated is eliminated. This implies that the packing and filling machine must also be kept sterile in that part where filling of the packaging containers takes place.

In today's food packing processes (the term 'food' is here taken to signify all types of solid and liquid foods, i.e. also juices, milk and other beverages), the packaging material or the ready-to-fill package is often sterilised by direct contact with a fluid, liquid or gaseous sterilisation agent. Packing processes are often continuous, high-speed processes of the 'form-fill-seal' type, i.e. processes in which packaging material in the form of a web or in the form of sheets is continuously fed through a machine, sterilised by passing through a liquid solution of a rapidly acting sterilisation agent or a gaseous medium, dried or ventilated with sterile air, formed to the desired geometric configuration in order to be filled, for example a beaker, blank or tube, filled with the food which is to be packed and sealed, all under sterile conditions. Bottles and beakers manufactured by means of various forming methods may also need to be sterilised in the same manner before being filled with their intended product contents. The direct contact stage may be put into effect by dipping the entire package or the packaging material down into the liquid sterilisation agent, as well as by spraying or coating the sterilisation agent on the packaging material, package wall or on the surfaces in a sterile space in a packing and filling machine, or alternatively by means of direct contact with a current of gaseous sterilisation agent. Since the sterilisation stage normally takes place prior to filling and sealing of the package in a high speed packing process, it is vital that the sterilising substance or agent can rapidly be dried off and removed from the packaging material before it is filled with the food product. On the other hand, it is of decisive important that there is sufficient sterilisation agent in the solution or the gas in order efficiently and rapidly to be able to exterminate all micro-organisms which exist on the packaging material. Those parameters which are vital to take into consideration for rational and sufficient sterilisation are hence the concentration of the sterilisation agent in the liquid or gaseous medium, the temperature of the sterilisation medium, as well as the temperature of the packaging material, and the time for contact between sterilisation medium and packaging material. These parameters must be balanced against the time which is required for drying or ventilating off the sterilisation medium from the packaging material and against the desired speed of the packing and filling process seen as a whole. The sterilisation agent which is most commonly used in food packing is hydrogen peroxide, since it is relatively economical, rapidly exterminates bacteria and micro-organisms and has been approved by the authorities for use in the food industry and thereby fills today's needs of the packaging industry.

A vital advantage in sterilising by means of a gaseous medium rather than a liquid medium is that a gas is lighter and more reliably penetrates into nooks and crannies in the packaging container or the filling machine, respectively. This is of particular importance in the sterilisation of filling and packing machines which often include many parts combined with difficult geometry.

Sterilisation by means of a gaseous medium can be put into effect in different ways. It is very common to lead in a gaseous medium of a vapour of the sterilisation agent to the space which is to be sterilised and then allow the gas to condense on the package or machine parts intended for sterilisation. However, the condensation method entails an extra step in which both machine and package parts must be dried and condensed vapour be ventilated off. In particular regarding certain types of plastics, especially, for example, polyethylene terephthalate (PET), hydrogen peroxide is adsorbed to the surface and is difficult to ventilate/dry off. This increases the risk of residual quantities of sterilisation agent, often hydrogen peroxide, in the packaging container. Such residues of the sterilisation agent can negatively affect the contents of the package, and the authorities have set up limits for such residual quantities which may not be overstepped. Also in machine spaces with small pockets and crannies, residual quantities of sterilisation agent may be unsuitable, for example since this may splash down into both contents and packaging containers.

According to an alternative method, the gaseous medium is heated, like the package or machine parts intended for sterilisation, to such a level that the medium is kept in the gas phase without condensing on the surfaces of the machine or package parts, respectively. While this method entails that the sterilisation surfaces must be heated up before the gaseous sterilisation medium reaches them, this may be preferable in many applications, ahead of condensation sterilisation, in particular if it is difficult to remove the condensate from surfaces in nooks and crannies of the packaging containers or the filling machine, respectively. According to this process, the packaging container or the filling machine is, during the sterilisation treatment, subjected during a given time to a gaseous sterilisation medium, at a specific temperature, a certain predetermined flow rate and a specific dew point. These parameters must be monitored in order to realise a reliable and efficient bactericidal effect. The dew point of the gaseous medium is the temperature to which the gas must be cooled for condensation to take place. This temperature depends upon the content of sterilisation agent in the gas and can be adapted to be employed for different environments for different sterilisation surfaces. Thus, the gaseous medium and sterilisation surfaces, respectively, should be kept at a minimum temperature so that the dew point of the gas is not reached and it is thereby possible to avoid condensation on the surfaces.

According to this latter non-condensation method, it is particularly important that the gas is of good quality, i.e. that it is totally vaporised and, as far as possible, free of liquid droplets and aerosols. When such droplets and aerosols are present, the risk increases that the gas will partly condensate when it impinges on the sterilisation surfaces, and will leave residues of, for example, hydrogen peroxide and stabilisers on surfaces downstream in the flow of the gaseous medium. Moreover, in many cases they impede an accurate measurement of the concentration of the sterilisation agent in the gas.

Consequently, stringent requirements are placed on the vaporisation process, and its monitoring. A plurality of different parameters must be placed in relation to one another in order to obtain complete vaporisation, at the same time as the concentration of the gaseous sterilisation agent must be kept constant between a given minimum level and maximum level. The parameters which interplay in the vaporisation process are, for example, pressure, temperature, flow rate and mixture ratio, i.e. concentration, between sterilisation agent and gas, normally hydrogen peroxide and air and water vapour.

As far as we know, there is no recognised system today for realising, in a reliable process, a completely vaporised sterilisation gas of high quality and uniform concentration. The known vaporisers which are available on the market today all function more or less satisfactorily, but none of them can guarantee continuous production of a sterilisation gas, preferably containing hydrogen peroxide, of uniform concentration and substantially free of aerosols.

Hitherto, the concentration of sterilisation agent, in particular hydrogen peroxide, in aqueous medium, has only roughly been estimated on mixing of the sterilisation agent with water, and thereafter measured only sporadically with the aid of outmoded laboratory methods. The consequence is that the sterilisation agent is inputted into the process more or less by rule of thumb and only roughly estimated as lying within the desired concentration range. With gaseous sterilisation medium, it is more critical that the concentration be carefully monitored and regulated, since the concentration is often relatively low, of the order of magnitude of approx. 5-20 g/m$^3$, and the stay time optimised so that the gas is kept in the sterilisation area for as a short a time as possible. If the concentration falls slightly, the risk of insufficient sterilisation increases considerably.

One object of the present invention is thus to realise a method of producing a gaseous sterilisation medium, which obviates or reduces the above-outlined problems.

A further object of the present invention is to realise a method of producing a gaseous sterilisation medium with regulation and monitoring so that the gaseous medium continuously has a uniform, predetermined concentration, and a minimum quantity of liquid droplets and aerosols.

**OUTLINE OF THE INVENTION**

These and other objects are attained according to the present invention by means of the method as described in appended Claim 1.

Preferred and advantageous embodiments of the method according to the present invention have further been given the characterising features as set forth in appended subclaims 2 to 6.

According to another aspect of the present invention, there will be realised a system and an apparatus for reducing the method according to the present invention into practice as defined in appended Claim 7.

Preferred and advantageous embodiments of the system according to the present invention have further been given the characterising features as set forth in appended subclaims 8 to 10.

A method according to the present invention thus comprises the steps of vaporising a liquiform medium containing the above-mentioned sterilisation agent, detecting aerosols and liquid droplets in the gaseous medium, continuously measuring the concentration of the agent in the gaseous medium, and continuously processing measurement signals from the above-mentioned detectors and measurement instruments by executing calculations and converting them into output signals for continuously regulating and monitoring the function of the vaporiser.

For vaporising, use is made of a vaporiser of conventional type. A common type of vaporiser functions such that liquiform sterilisation agent is sprayed into the vaporisation chamber together with a warm air current which spreads and finely-divides the liquid droplets. Vaporisation takes place in that the spray-gas mixture is led past heater elements which heat up the gas mixture and vaporise substantially all of its liquid content. Another types functions in that the liquid droplets are sprayed in direct against hot metal surfaces so that the droplets are vaporised and then mixed with a regulated heated air current. The vaporiser may be of any known design and construction whatever, vaporisation by tube or plate heat exchange, admixture with hot air, by means of different designs of nozzles, etc.

Aerosols can be detected by means of, for example, light absorption spectophotometry or light scattering methods. Non-optic detectors are also conceivable for this purpose which act by means of acoustic methods, for example based on ultrasound. Preferably, light absorption is employed to detect aerosols and liquid droplets in the gas.

The concentration of an agent in a gas can be determined more or less accurately using different optic or non-optic methods. Among the non-optic methods, mention might be made of conductive measurement methods or electro-chemical measurement methods. According to the invention, the concentration of the gaseous sterilisation medium is measured by means of light absorption spectrophotometry, since this method gives the requisite high level of accuracy and, moreover, can be automatically combined with detection of aerosols and other disruptive matter, and compensate for the action of them on the measurement results.

The measurement signals from the aerosol detector and the concentration metre are inputted in a calculator unit in which calculation and conversion take place for obtaining the output signals which control how the various parameters in the vaporiser are to be regulated. The calculator unit suitably comprises a microprocessor or a so-called CPU-unit.

According to one preferred embodiment of the present invention, the method also includes a step in which such gas as should not reach the sterilisation surfaces may be removed during a brief period, while the function of the vaporiser is regulated so that the gas will have the correct concentration and will be completely vaporised again, i.e. is free of aerosols and liquid droplets. Such a step is suitably carried out with the aid of a bleeder valve and bleeder conduit to a receptacle vessel for 'dumped' gas, which can be connected in when the values of the concentration deviate excessively from the norm value or when aerosols are detected in the vaporised gas.

According to another preferred embodiment of the present invention, a step is also included in which liquid droplets and aerosols are continuously removed from the gaseous medium. This may suitably be put into effect using a hot filter of some type which can break up and finely-divide the small liquid particles at the same time as they are heated up so that they are totally vaporised.

According to yet a further preferred embodiment of the present invention, aerosols are detected in the same step and with the same apparatus as the measurement of the concentration of the sterilisation agent. Such an apparatus is preferably a metering apparatus which is based on UV-absorption spectrophotometry in accordance with the following.

Light absorption spectrophotometry, and in particular UV-absorption spectrophotometry, is highly suited for conducting quantitative analysis of a light-absorbing substance or agent in a sample medium since the light absorption of a substance is directly dependent upon its concentration, i.e. that the concentration of the substance is in inverse proportion to the height of the peaks which are plotted from the output signal of a light intensity detector. Moreover, light absorption methods are relatively easy to carry out, rapid, reliable, reproducible and accurate.

All substances in solution or gas phase absorb radiation at different characteristic wavelengths within the electromagnetic spectrum. In particular, most substances absorb UV-light in the UV region of the spectrum.

It is normally understood that UV-light extends from approx. 10 to approx. 400 nm, while visible light extends from approx. 400 to approx. 750 nm. The UV region is divided into the UVA-, UVB- and UVC spectrum. UVA extends from approx. 320 to approx. 400 nm, UVB from approx. 280 to approx. 320 nm and UVC from approx. 200 to approx. 280 nm. Chemical UV analyses are normally carried out at wavelengths lower than 160 nm. At wavelengths shorter than 220 nm, it is, however, necessary to conduct the analyses under oxygen-free conditions, i.e. in the absence of air or water, since otherwise the absorption of the oxygen would disrupt the measurement results and since oxygen dissociates and forms ozone on irradiation (which would also disrupt the measurements).

According to traditional light absorption analysis methods, in particular the absorption of UV-light, the substance or agent which is to be measured is dissolved or vaporised in a medium which absorbs much less at the same characteristic wavelength than the substance or agent itself. The intensity of the light which is allowed through the sample medium which contains the agent to be measured is detected, like the light which is allowed through a reference sample of the medium which does not contain the agent under examination at the same characteristic wavelength. The two output signals which represent the light intensity are employed for calculating the concentration of the agent in accordance with Beer-Lambert's Law:

$$\text{Log } l_0/l = A \text{ (=Absorbency)} = \varepsilon \, LC \quad \text{i.e. } l/l_0 = 10^{-\varepsilon LC}$$

Where 1 and $l_0$ are the intensities of the light allowed through the sample and reference sample, respectively, $\varepsilon$ is the absorption coefficient of the specific substance or agent at a specific wavelength in a specific medium at a predetermined temperature, L is the length of the measurement distance through the sample which is to be measured and C is the concentration of the substance in the sample. Since the length of the measurement distance, i.e. the length of the measurement cell if such is employed, is a quantifiable constant, and since absorption coefficients, $\varepsilon$, for different substances and media are well known and documented at different wavelengths, the light intensities 1 and $l_0$ may be continuously measured and thereby also the concentration of the substance or agent in the medium can be continuously measured. Beer-Lambert's Law applies to all monochromatic light, i.e. light of a specific wavelength which has a narrow spectral band width.

Calibration may be put into effect, for example, by measuring the light emitted and allowed through a reference sample. Normally, such a reference measurement may be carried out either in the same vessel or measurement cell which contains the sample medium, by regularly flushing the measurement cell with the gaseous or liquiform medium which does not contain the substance or agent to be measured, or alternatively by measuring the reference sample in another identical measurement cell. A disadvantage in measuring through two different measurement cells is, however, that it may be difficult to be certain that both of the measurements are carried out under exactly the same conditions.

In all types of gaseous sterilisation media, small liquid droplets or aerosols can, however, be formed, which would also disrupt the measurement results.

Japanese Patent Application JP-A-01244341 describes a method and an instrument for measuring the concentration of ozone in a fluid medium by measuring the absorption at two wavelengths, where the fluid medium also contains disruptive light-absorbing substances, such as chlorine, sulphur dioxide or nitrogen oxide. According to one embodiment, the first wavelength is 254 nm, at which both ozone and the other substances absorb, while the second wavelength is 184.9 nm, at which only the other substances absorb light. Measurements at a wavelength of 184.9 nm however restrict the type of sample medium to such as neither contain air nor water nor moisture, since oxygen reacts so that ozone is formed on the action of UV radiation at such short wavelength. This will inevitably disrupt the measurements if the substance which is to be measured absorbs light at the same wavelength region as ozone.

According to a second embodiment, the first wavelength is 254 nm, while the second wavelength is 436 or 546 nm, or both wavelengths are measured as a second and third wavelength.

The double wavelength method, i.e. the method of measuring the light absorption at two different wavelengths still does not guarantee that the desired accuracy for certain measurement applications is attained. The light source which caters for the light which is to be transmitted through the sample medium does not transmit the same quantity of light at different points in time. Normally, the light intensity declines with the age of the light source, but in addition the intensity varies with variations in the electric system and the current power supply. JP-A-01244341 discloses that it is possible, according to known methods, also to measure the intensity of the light which is emitted direct from the lamp before being transmitted through the sample when measuring at only one wavelength, for the purpose of compensating for variations in the radiation of the lamp.

However, in JP-A-01244341, it is assumed that variations in the intensity of the light emitted from the lamp are eliminated by measuring at different wavelengths, since the intensity deviation would probably the same at both wavelengths.

However, this is not true, and can only be assumed for certain purposes when high accuracy is not required and when the two wavelengths are selected from a narrow band of the spectrum, i.e. lie relatively close to one another. We have found that for the purpose of exactly determining the concentration of a substance or agent in a medium, it is necessary to compensate for variations in the lamp at the first wavelength as well as at the second. For our purpose, it is desirable to determine concentrations with an accuracy of as high as $\pm 3\%$, preferably 2%.

While it is generally known to measure the concentration of different substances in a liquiform or gaseous medium using absorption spectrophotometry, it is hitherto unknown to measure the concentration of a sterilisation agent in a sterilisation medium in connection with processes for sterilising packaging material for packing foods, using light absorption methods. There are no methods or instruments available on the market today for measuring the concentration of substances, such as, for example, hydrogen peroxide, with sufficient accuracy using light absorption spectrophotometry in that type of sterilisation medium which is employed in the sterilisation of packaging materials.

Further, there has not been any system available for producing a gaseous sterilisation medium which is continuously regulated and monitored so that the gas maintains a predetermined concentration and is substantially totally free of aerosols and liquid droplets.

US Patent No. 5,872,359 discloses a method for the production of a gaseous medium containing a sterilisation agent and for regulating and monitoring the concentration and quality of the sterilisation medium.

US Patent No. 4,296,068 relates to sterilisation in food packaging and mentions the importance of avoiding large drops of liquid to avoid condensation of the sterilisation "mist" in the sterilisation assembly.

By selecting the first wavelength from the UV spectrum and the second wavelength from the visible spectrum, it is possible to compensate for the disruptive absorption from disruptive matter, such as, for example, dust particles or condensation droplets, aerosols, in the most efficient possible manner. Those substances which are to be measured are often broad band UV-absorbing substances such as, for example hydrogen peroxide, which however absorbs substantially less light, or no light at all, in the visible spectrum. On the other hand, the above-mentioned type of disruptive matter absorbs substantially the same quantity of light in the UV region and in the visible spectrum.

The first wavelength or wavelengths are selected from between approx. 220 nm and approx. 320 nm, since this region lies sufficiently far away from the visible spectrum, and since the type of broad band absorbing substances which is often employed have their absorption maximum within this wavelength region. By measuring at wavelengths where the substance or agent has adequate and sufficiently strong absorption, a higher degree of accuracy can be attained. Of major importance also is at what wavelengths the light source emits light of sufficiently high intensity. The most preferred light source of those available on the market today is the low pressure mercury lamp which has a powerful light emission at 254 nm, more precisely at 253.7 nm. Consequently, the most preferred first wavelength is selected at approx. 254 nm. Another more moderate light irradiation takes place at approx. 313 nm, which may also be preferred for certain applications.

The second wavelength or wavelengths should be measured at wavelengths of approx. 385 nm and longer, more preferably among wavelengths at between approx. 400 nm and approx. 700 nm and most preferably from approx. 436 nm and/or approx. 546 nm.

Calibration is carried out by measurement through a reference sample containing the same fluid medium as the measurement sample, but none, or substantially less of the substance or agent which is to be measured. By measuring the intensity of permeated light through samples as well as reference samples at the first, as well as the second wavelength (s), and by employing the obtained values in Beer-Lambert's relationship, light absorbency of the sample can be determined at each wavelength.

As was explained above, the calibration measurement can take place in another but identical measurement cell, containing only reference sample, or alternatively in the same reference cell, but at another point in time. The latter method is to be preferred, since it gives greater reliability against differences in the sample flows and against differences between two different measurement cell windows.

The method functions particularly well for broad band UV-absorbing substances which do not absorb, or absorb considerably less light in the visible spectrum. Most suitably, the concentration of hydrogen peroxide can be measured by the method according to the present invention since these substances have such broad band UV-absorption properties. Hydrogen peroxide is also the most generally employed sterilisation agent in the food and packaging industries.

In the food packaging industry, the sterilisation agent is most often enveloped in a fluid, liquiform or gaseous medium, containing water, moisture and/or air. Preferably, the medium is a mixture of air and a gaseous vapour of the sterilisation agent. Air and water vapour are preferred since they are harmless media, from both the environmental and the food hygiene aspects.

For light absorbency measurements of gaseous hydrogen peroxide, the first wavelength is most advantageously selected from approx. 254 nm. The length of the measurement distance is preferably from approx. 10 to approx. 250 mm depending on the concentration in the sample medium, in the absorbency measurement of gaseous hydrogen peroxide.

As was mentioned above, the light source is advantageously of the type which emits light at wavelengths from between approx. 220 nm and approx. 320 nm, both as light of another wavelength or a second wavelength region of approx. 385 nm and longer. Most preferably, a light source for such wavelengths is a low pressure mercury lamp.

According to the present invention comprises an apparatus for concentration determination which includes a light source, a measurement distance (L), a measurement device in the form of detectors which produce detector output signals, and a calculator unit for calculating the actual concentration with high accuracy by applying Beer-Lambert's relationship to the output signals. The calibration measurement is carried out with the aid of detectors which detect that light which has been transmitted through a reference sample, containing the same medium as the measurement sample, but substantially less of the substance or agent which is to be measured, the detectors being adapted to measure the light intensity at the first and second wavelengths, respectively. By measuring the intensity of the light which has been transmitted through the sample as well as through the reference sample at the first as well as at the second wavelengths, and by employing the obtained values in Bee-Lambert's relationship, the light absorbency in the sample can be determined at each one of the first and second wavelengths.

In concentration determination of hydrogen peroxide in a gaseous medium, the length of the measurement distance is preferably from approx. 10 to approx. 250 mm and the first and third detectors are adapted to measure the light intensity at approx. 254 nm.

The method and the system for producing the gaseous medium and monitoring its concentration and gas quality according to the present invention are particularly suitable for use for the sterilisation of packing and filling machines and packaging materials in the food packaging industry, since it affords a high degree of accuracy in the concentration measurement despite the presence of light-absorbing disruptive matter in the sterilisation medium and in such a manner makes for more reliable sterilisation and a lower risk of residues of the sterilisation agent (because of surplus of sterilisation agent) in the sterilised packages, as well as a more efficient use of the sterilisation agent, and at the same time ensures that no sterilisation medium condenses on the sterilisation surfaces.

## DETAILED DESCRIPTION OF PREFERRED EMBODIMENTS

Further advantages and preferred characterising features of the method and the apparatus according to the present invention will be clarified in the following description, with particular reference to the accompanying Drawings.

Even though the present invention will hereafter be described with specific reference to a method and a system, it should nevertheless be observed that the present invention, in its broadest scope of protection, is not restricted exclusively to this practical application but has been selected as examples among many other conceivable apparatuses for reducing into practice the method according to the present invention as defined in the appended Claims.

In the accompanying Drawings:

Fig. 1 schematically illustrates a system according to two alternative embodiments of the present invention for producing a gaseous medium containing a sterilisation agent, such as, for example, hydrogen peroxide; and

Figs. 2 and 3 each illustrate schematically an apparatus for monitoring the concentration of a light-absorbing substance; the apparatus being included in the system for producing gaseous sterilisation medium.

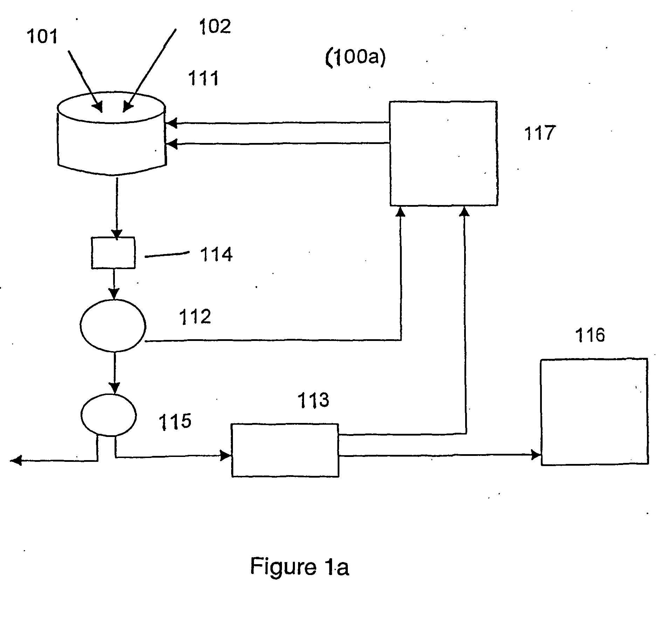

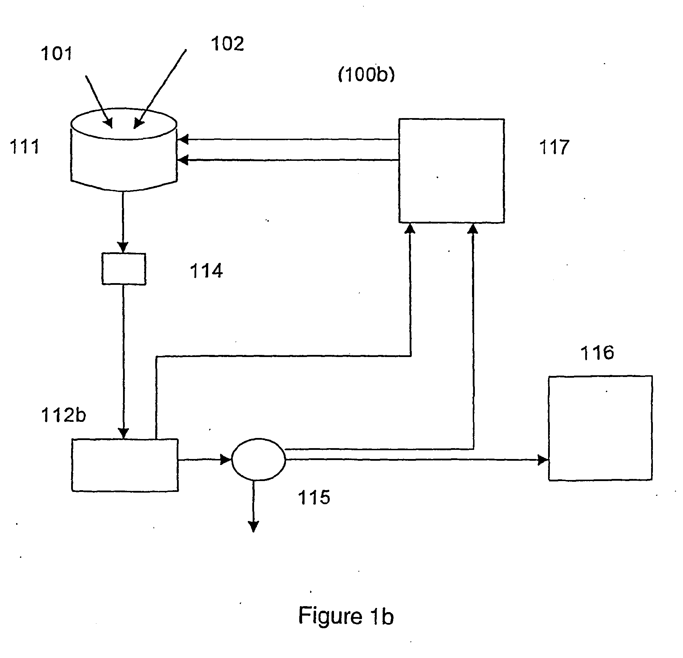

Referring to the Drawings, Fig. 1 thus shows a system 100a comprising a vaporisation device 111. A current of liquid sterilisation solution 101 is sprayed into the vaporiser together with a current of hot air 102. The vapour which is produced in the vaporiser is led further through a heated gas conduit, in order to prevent the gas from condensing, and is caused to pass an aerosol detector 112 for verifying the quality of the gas. Thereafter, the gas is led further to a concentration metre 113 for continuous measurement of the concentration of the gaseous sterilisation medium. According to one preferred embodiment, an aerosol filter 114 may be connected into the system direct after the vaporisation device, if necessary, or alternatively always be permanently connected in so as to ensure a substantially aerosol-free gas. According to another preferred embodiment, the system has a bleeder valve and bleeder conduit 115 for gas which has been found to contain aerosols, located after the aerosol detector 112 so that gas of poor quality can be led off from the system without passing either the concentration metre or the sterilisation surfaces and the sterilisation space 116. The output signals from the aerosol detector and concentration metre 113, respectively, are transmitted to a calculator unit 117 which, on the one hand, calculates the concentration of the gaseous sterilisation medium and, on the other hand, compares the values of the concentration of the gas, possible content of aerosols, as well as the temperature, pressure and flow rate of the gas with the predetermined norm values which the gas should maintain for a satisfactory sterilisation to be able to take place. One or more regulator signals are then transmitted from the calculator and regulator unit back to the vaporiser device for correction of the parameters which control its function, i.e. flow rates, mixing ratios, temperature and pressure. Principally, the concentration of the gas is regulated by adjusting the flows of the liquiform sterilisation agent and hot air in to the vaporiser. Since hydrogen peroxide in practice disappears and is broken down on the way through the conduit up to the concentration metre and the sterilisation space, the actual concentration is always lower than the theoretical. By now comparing the actual value from the concentration metre with the norm value, automatic and continuous regulation of the flows in to the vaporiser device can take place, and thereby the norm value can be attained even at the metre and sterilisation space.

Fig. 1b shows a more preferred system 100b according to the present invention.

Gaseous sterilisation medium is produced in a vaporiser device 111. A current of liquiform sterilisation solution 101 is sprayed into the vaporiser together with a current of hot air 102. The vapour which is produced in the vaporiser is led further through a heated gas conduit, in order to prevent the gas from condensing, to a combined apparatus for aerosol detection and for continuous measurement of the concentration 112b of the gaseous sterilisation medium. According to a preferred embodiment, an aerosol filter 114 may be connected into the system direct after the vaporisation device, if necessary, or alternatively always be permanently connected in so as to ensure a substantially aerosol-free gas. According to another preferred embodiment, the system has a bleeder valve and bleeder conduit 115 for gas which has been found to contain aerosols, located after the aerosol detector and the concentration metre 112b so that gas of poor quality can be led off from the system without passing to the sterilisation surfaces and the sterilisation space 116. The output signals from the aerosol detector and concentration metre 112b are transmitted to a calculator unit 117 which, on the one hand, calculates the concentration of the gaseous sterilisation medium and, on the other hand, compares the values of the concentration of the gas, possible content of aerosols, as well as the temperature, pressure and flow rate of the gas with the predetermined norm values which the gas should maintain for a satisfactory sterilisation to be able to take place. One or more regulator signals are then transmitted from the calculator and regulator unit back to the vaporiser device 111 for correction of the parameters which control its function, i.e. flow rates, mixing ratios, temperature and pressure. Thus, the system loop combines aerosol detector and concentration metre in the same apparatus and the same steps in the method.

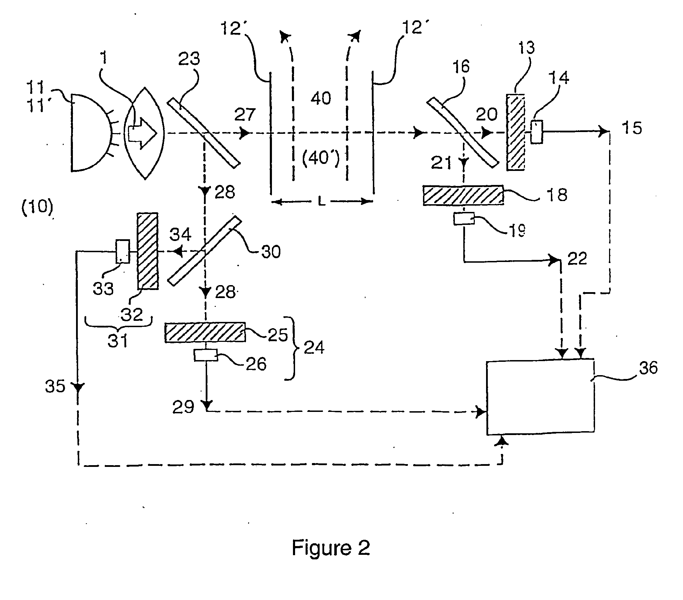

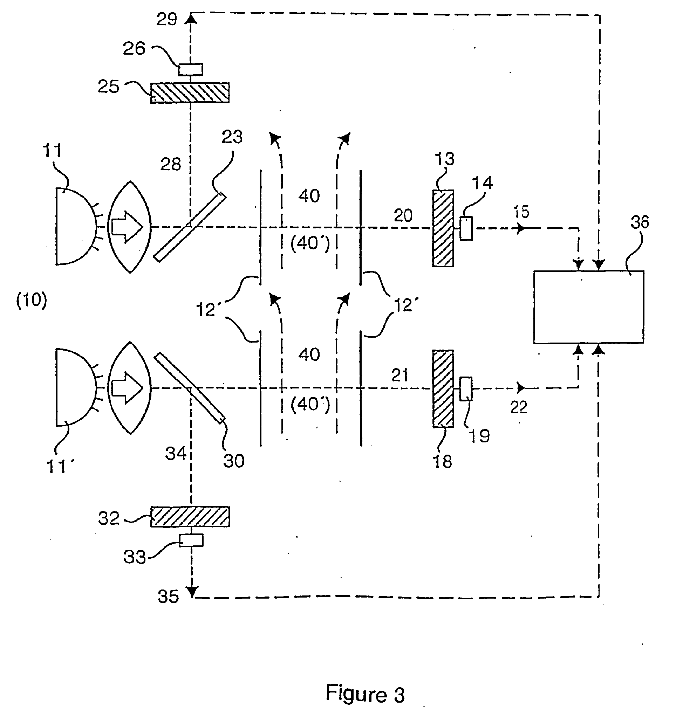

Figs. 2 and 3, respectively, show a variation of the design and construction of the concentration metre which is encompassed in the system according to the invention and which also functions as an aerosol detector.

The concentration of substances that have broad absorption bands in the UV spectrum, but with a light absorption at wavelengths longer than 385 nm which is close to zero can be measured and regulated using the method and the system according to the present invention. One typical such substance is hydrogen peroxide.

For the purpose of catering for light of the suitable wavelengths, one or more light sources (11) are provided.

The light source according to the present invention are such as produce light at wavelengths which extend from shorter UV wavelengths from UVB and UVC, i.e. from approx. 220 to approx. 320 nm as well as light from the visible wavelength region longer than approx. 385 nm. Examples of such light sources are lamps of the gas discharge type which give broad spectrum light, e.g. a xenon lamp or alternatively high or low pressure mercury lamps. However, apparatuses UV lasers may also be employed according to the preferred embodiment of the invention. It is, for example, possible to provide UV light of one or more predetermined wavelength(s) with one or more laser diodes. In order to give light from the visible spectrum, an additional light source for visible light must then be provided.

For light of other wavelengths, other well-known light sources may be employed.

The most preferred light source is a low pressure mercury lamp of the type which is commercially available today, since it can give a spectral line from the UV region at a predetermined wavelength of minimal band width and high intensity, as well as light from the visible spectrum. The relevant UV wavelength is approx. 254 nm, or more precisely 253.7 nm, and another distinct spectral line is to be found at approx. 313 nm. Light of other wavelengths can also be filtered off from the beam of the UV light source. If desired, as may, for example, be the case regarding a low pressure mercury lamp, light emitted from the lamp can be centred along the path of the light beam with a collimating lens (1). UV light and visible light with at least two different sources (11, 11') or with only one and the same light source (11).

The measurement cell or the monitoring space (12) containing the gas or liquiform sample which is to be measured has windows (12') of quartz glass or similar optically weft-functioning transparent material. The sample medium which is to be measured is preferably measured while flowing through a measurement cell. For substances which can be influenced by UV radiation, such as hydrogen peroxide, it is desirable to measure the concentration in a flowing sample medium.

In a packing and filling machine which employs gaseous sterilisation medium, the measurement apparatus may advantageously be built around a conduit for transferring the gas current into the sterilisation zone. The walls of the conduit are then provided with windows (12'), for example made of quartz glass, in order to be able to allow light to pass through from the light source, outside the wall of the conduit, into the gas current and further through a window (12') in the opposing conduit wall, over to each respective light detector. A separate measurement cell located outside the normal conduit, and in a separate loop for providing the measurement cell with sample medium is probably then not necessary. By measuring direct in the current of sample medium, a simpler construction of the apparatus on installation in a filling machine is possible. Instead of measuring through a measurement cell, the light absorption can, by such means, be measured through a measurement space. The distance between the two quartz glass windows constitutes the length (L) of the measurement distance. In particular in cases involving a warm gaseous medium, a separate measurement loop causes problems in the form of condensation droplets on the windows of the measurement space. In a sterilisation apparatus, the measurement space may even consist of the sterilisation chamber proper. Quartz windows or the like may then be located on the opposing walls of the chamber.

The sample medium (40), i.e. the sterilisation medium, or a reference medium containing none, or substantially less, of the sterilisation agent (40'), flows through the measurement cell at such a speed that the light cannot act on the sterilisation agent.

The medium may be any liquiform or gaseous medium whatever which does not disrupt the light absorption measurements according to the invention. Normally, sterilisation media are based on water or sterile air or hot vapour containing air. However, other alternatives are conceivable, such as, for example, a pure, inert gas such as nitrogen, or a sterilising solution which would not be harmful to the packed product or act as a safety risk in the packing process environment. The first and second wavelengths should preferably be selected so that the substance or agent which is to be measured absorbs a sufficient quantity of light at the two wavelengths. The medium proper should preferably absorb considerably less light, or none, at the same wavelengths as the agent which is to be measured.

The length (L) of the measurement cell or the measurement distance through the sample medium, i.e. the distance which the light is transmitted through the sample medium, can be selected in accordance with the desired measurement range, i.e. the concentration range in which measurement is to be made, the specific medium and the specific measurement wavelength.

The measurement distance (L) has a first end at which the light source is located and a second end, on the opposite side of the measurement space from the light source, at which a device for detecting light which has been transmitted through the sample medium is located.

Consequently, with a view to detecting and measuring light which has been transmitted through the sample medium, first and second detectors (14, 19) are located on the opposite sides of the measurement cell at the second end of the measurement distance. The first detector (14) is preferably adapted to detect UV light at at least one predetermined first wavelength. Any standard detector whatever, preferably adapted to measure wavelengths of 220-320 nm is suitable, for example a UV-sensitive photo diode.

In order to limit the light which is transmitted through the sample to the selected, predetermined measurement wavelengths, and by such means prevent disrupting light from other diffuse wavelengths from entering into the detector, it may be advantageous to place an optic filter (13) in front of the first detector (14) along the path of the light beam. Such optic filters for UV light can advantageously be of the band pass filter type. In the case when the light source radiates light of only one distinct wavelength or a distinct wavelength region, such as a laser diode, an optic filter may be dispensed with. An optic filter may also be unnecessary if the detector has the spectral sensitivity range which is required.

The second detector (19) is preferably adapted to detect light at a predetermined second wavelength or wavelength region from the visible spectrum i.e. wavelength(s) longer than approx. 385 nm, more preferably within the range of from approx. 400 nm to approx. 700 nm. The second detector (19) is suitably a photo diode and has an optic filter (18) of the visible "cut-off" or "cut-on" filter type, with a view to filtering off all UV light and only allow through visible light. In particular in the use of a low pressure mercury lamp, a second detector is preferred which is adapted for measuring light of 436 nm and/or 546 nm, since it produces well-defined spectral lines at these wavelengths.

The light which was transmitted from the light source may thus be transmitted through the sample medium via a single light beam containing light of several different wavelengths, both from the UV spectrum and the visible spectrum (see Fig. 2), as, for example, is the case with a mercury lamp. The light with the different wavelengths may also be provided by two or more light sources and then gathered into a single common light beam, with the aid of known optic devices (mirrors, reflectors). Alternatively (see Fig. 3), light may be transmitted via two separate light beams, one for measuring at the first predetermined UV wavelength by means of first detector, and another for measuring at the second predetermined visible wavelength or wavelength region, with the aid of a second detector. In the latter case, uncertainty may arise in that the light beam passes through different parts of the sample medium, or even through different measurement cells, and in that the quantity of disruptive matter may be different (dust, aerosols, droplets, particles, etc.). The first case, i.e. with the light source or light sources which give a single light beam is thus preferred according to the present invention. For detecting light of two separate wavelengths, the main light beam may be divided into two light beams once the light has passed through the sample medium. This is preferably realised by means of a beam splitter (16) placed at the second end of the measurement distance, along the path of the light beam before the detectors (14, 19) and, where applicable, the optic filters (13, 18). Such a beam splitter may be a mirror or a so-called beam splitter cube or other type of optic window which is designed to allow through a part of the light and reflect the other part of the light.

The beam splitter (16) thus splits up the light beam into two separate light beams (20, 21), which by such means provide the first and second detector, respectively, with light. The first light beam (20) which gives light to the first detector (14) preferably passes through a first optic filter (13) before reaching the detector, with a view to limiting the light which enters into the detector to the predetermined first wavelength(s). Correspondingly, the second light beam (21) preferably passes through a second optic filter (18) with a view to limiting the light which enters into the second detector (19) to the predetermined second wavelength(s).

Consequently, by directing a light beam from the light source through a sample with the liquid medium (40) along a measurement distance of the length (L), containing the agent which is to be measured, as well as disruptive matter, it is possible to detect the intensity of light transmitted (20) through the sample medium (40) at the first wavelength, and also direct light from the light source through the reference sample (40'), which contains none, or substantially less, of the agent which is to be measured, along a measurement distance of the same length (L), and then detect the intensity of light which has been allowed through (20') at the first wavelength through the reference sample (40'), first detector output signals (15, 15') are produced for indicating the difference in intensity of the light allowed through the sample and the reference sample, respectively. By applying Beer-Lambert's relationship to the relative values of the output signals, the concentration of the light-absorbing agent can normally be determined. According to the present invention, the actual concentration of the agent is determined by correction, by employing the corresponding second detector output signals (22, 22') from the same measurements at the second wavelength, in order to eliminate the influence of impurities in the sample (40).

The analogous detector output signals (15, 22) are transmitted to a converter unit for conversion into digital signals and are then passed further to a calculator unit (36) for calculation and evaluation of the concentration according to Beer-Lambert's relationship. Where applicable, the output signals can be processed for further realising input signals to an automatic concentration regulator system for controlling the dosing of the agent to the liquiform or gaseous medium. In order to be able to apply Beer-Lambert's relationship, the intensity of light transmitted through the sample medium, as well as through the reference sample should measured, i.e. the sample which is free, or substantially free, of the agent which is to be measured. As was previously mentioned, this may preferably be put into effect either by changing contents from sample to reference sample in one and the same measurement cell now and again. Alternatively, the reference sample may be measured in a separate measurement cell only with the liquiform or gaseous medium (free of the sample agent). The first case is preferred since it gives the greatest reliability and accuracy.

The calculations in the calculator unit are preferably carried out as follows:

1) Traditionally, the concentration is determined with the aid of Beer-Lambert's relationship, i.e.

$$C = 1/\varepsilon\, L * \log (I_{uv(0)}/ I_{uv})$$

Where $I_{uv(0)}$ is the intensity of UV light allowed through the reference sample, i.e. only the medium U(40') at the first predetermined UV light wavelength (the first detector output signal 15'), and is the intensity of light allowed through the sample medium, i.e. the medium containing the agent which is to be measured as well as disruptive matter (40), at the first predetermined UV wavelength (the first detector output signal (15).

2) However, according to the present invention, the intensity of the permeated light varies also because of disruptive matter in the liquiform or gaseous medium, such as dust, aerosols and other more or less solid particles. Consequently, the relationship $I_{uv(0)}/ I_{uv}$ must be corrected with the relationship $(I_{vis(0)}/I_{vis})$ where $I_{vis(0)}$ is the intensity of light allowed through the reference sample, i.e. only the medium (40'), at the second predetermined visible wavelength (second detector output signal (22'), and $I_{vis}$ is the intensity of light allowed through the sample medium, i.e. the medium containing the agent which is to be measured as well as any possible disruptive matter (40) at the second predetermined visible wavelength (the second detector output signal 22).

Consequently, the following applies:

$$C = 1/\varepsilon\, L * \log ((I_{uv(0)}/ I_{uv})\,(I_{vis}/ I_{vis(0)}))\,.$$

i.e.

$$C = 1/\varepsilon\, L * \log (I_{vis}/ I_{uv}) - 1/\varepsilon\, L * \log (I_{vis(0)}/ I_{uv(0)}).$$

Where the second term is determined on calibration and measurement through the reference sample and may then be stored in the calculator unit as a constant value. The relationship $(I_{vis}, / I_{uv})$ is thus measured continuously.

3) According to the present invention, the intensity of the light transmitted from the lamp but not transmitted through the sample medium is also measured and passed on as detector output signals to the data processing unit. The intention here is to compensate for the fact that the intensity of the light transmitted from the lamp may vary in time because the lamp ages or because of variations in the current power supply to the lamp. Such measurements may prove necessary, depending upon the quality of the lamp but also on the use of and purpose of the measurement and requirement on desired accuracy in the measured concentration. It has proved to be most desirable for the purpose of measuring the concentration on the sterilisation of packaging material, packages or equipment intended for food packing.

4) The relationship $(I_{uv(0)} / I_{uv})$ should hence be adjusted with the relationship $I_{uvref(0)} / I_{uvref}$ where $I_{uvref(0)}$ is the intensity of UV light transmitted from the light source at the first predetermined UV light wavelength at that point in time when the reference sample is measured (third detector output signal 29') and $I_{uvref}$ is the intensity of light transmitted from the light source, at the first predetermined UV wavelength at that point in time when the sample medium is measured (third detector output signal 29).

For certain purposes, it may be assumed that the intensity of the light transmitted from the light source varies with time as much at different wavelengths. However, such an assumption is, for most light sources, not true and causes poorer accuracy in calculations of the concentration. In the case with a low pressure mercury lamp and requirements on high accuracy, such as $\pm 3$ preferably $\pm 2\%$, in the concentration measurements, no such assumption is recommended. Once again, this of course depends on the circumstances and intentions with the measurements. In particular, changes in the environment around the measurement apparatus, such as temperature changes, also have different effects at different wavelengths on the function of the lamp and the light intensity. The relationship between the light intensities at different wavelengths thus varies with changes in the ambient temperature. Temperature fluctuations are common in environments for packing and filling machines.

5) Consequently, for improved accuracy in the measurements, the intensity of the light transmitted from the light source should be measured at the first as well as at the second predetermined wavelengths. Hence, the relationship $(I_{uv(0)} / I_{uv})$ should further be adjusted with the relationship $(I_{visref(0)} / I_{visref})$ where $I_{visref(0)}$ is the intensity of light transmitted from the light source at the second predetermined (the fourth detector output signal 35') at that point in time when the reference sample is measured, and $I_{visref}$ is the intensity of the light transmitted from the light source, at the second predetermined wavelength at that point in time when the sample medium is measured (fourth detector output signal 35).

6) Consequently; the concentration can be calculated as

$$C = 1/\varepsilon\, L * \log\left((I_{uv(0)} / I_{uv})\,(I_{vis} / I_{vis(0)})\,(I_{uvref} / I_{uvref(0)})\,(I_{visref(0)} / I_{visref})\right)$$

i.e.

$$C = 1/\varepsilon\, L * \log\left((I_{uvref} / I_{uv})\,(I_{vis} / I_{visref})\right) -$$

$$1/\varepsilon\, L * \log\left((I_{uvref(0)} / I_{uv(0)})\,(I_{vis(0)} / I_{visref(0)})\right)$$

where the second term is determined on calibration and measurement through the reference sample and can then be stored in the calculator unit as a constant value. Hence, only $I_{uvref}$, $I_{uv}$, $I_{vis}$, and $I_{visrer}$ need be continuously measured.

Accuracy in the concentration measurements according to this preferred embodiment is $\pm 3\%$, preferably $\pm 2\%$ which is desirable in processes for sterilisation of packaging material.

Compensation and correction calculations should be made for variations in the ambient pressure and temperature.

Consequently, with reference to Fig. 2, the apparatus may further include a second beam splitter (23) and a third detector device (24) including a third optic filter (25) and a third detector (26), the beam splitter (23) splitting up the light from the light source into a first light beam (27) and a third light beam (28) and being placed between the light source (11) and the first end of the measurement distance (L), the main light beam (27) being directed through the sample medium along the measurement distance, the third detector device (24) being designed to measure UV light at the above-mentioned first wavelength and being placed along the third light beam (28) and by such means giving a reference output signal (29) (corresponding to $I_{uvref(0)}$ and $I_{uvref}$, respectively) in order to compensate for fluctuations in the intensity of the light allowed through from the light source at the above-mentioned first wavelength. The third optic filter and the detector are preferably identical to the first optic filter (13) and the detector (14). The apparatus then further includes a third beam splitter (30) and a fourth detector device (31), including a fourth optic filter (23) and a fourth detector (33), the third beam splitter splitting off a fourth light beam (34) from the third light beam (28) and is placed between the second beam splitter (23) and the third and fourth detector devices, the fourth detector device (31) being designed to measure light of the second wavelength and being placed along the fourth light beam (34), and by such means giving a reference output signal (35) (corresponding to $I_{visref(0)}$ and $I_{visref}$, respectively) in order to compensate for fluctuations in the intensity of the light allowed through from the light source at the second wavelength.

The fourth optic filter and the detector are preferably identical to the second optic filter (18) and the detector (19). There will thus be obtained third and fourth detector output signals (29; 35) which represent the intensity of light emitted from the lamp at a certain point in time.

In an alternative apparatus, two light beams are directed through two discrete but identical measurement spaces (12), according to Fig. 3, which employs the same reference numerals for corresponding phenomena.

In Fig. 3, the light source (11), or, where applicable, the light sources (11, 11') produce two main light beams (20, 21) each one of which is to be transmitted through a measurement cell (12) or through different measurement spaces (12), both containing the sample medium and both with the same length of measurement distance (L). At the second end of the measurement distance in the first measurement cell, along the light beam (20), a first detector (14) is placed for detecting the intensity of the light allowed through at the first wavelength. Normally, the light first passes through a first optic filter (13) in order to limit that light which is to be detected to light only of the first wavelength. Correspondingly, at the second end of the measurement distance in the second measurement cell, along the light beam (21), a second detector (19) is placed for detecting the intensity of the light allowed through at the second wavelength. Normally, the light first passes through a second optic filter (18) in order to limit the light which is to be detected to light only of the second wavelength.

According to the preferred embodiment of the invention, the apparatus may then further include a first beam splitter (23) and a third detector device (24) including a third optic filter (25) and a third detector (26), the beam splitter (23) splitting up the light from the light source into a main light beam (20) and a third light beam (28) and being placed between the light source (11) and the first end of the measurement distance (L), the main light beam (20) being directed through the sample medium along the measurement distance, the third detector device (24) being designed to measure UV light at the above-mentioned first wavelength and being placed along the third light beam (28) and by such means giving a reference output signal (29) in order to compensate for fluctuations in the intensity of the light allowed through from the light source at the above-mentioned first wavelength. The third optic filter and the detector are preferably identical to the first optic filter (13) and the detector (14). The apparatus may then further include a second beam splitter (30) and a fourth detector device (31), including a fourth optic filter (32) and a fourth detector (33), the second beam splitter (30) splitting off a fourth light beam (34) from the second light beam (21) and is placed between the light source (11) and the second measurement cell, the fourth detector device (31) being designed to measure light at the second wavelength and being placed along the fourth light beam (34), and by such means giving a reference output signal (35) in order to compensate for fluctuations in the intensity of the light allowed through from the light source at the second wavelength. The fourth optic filter and the detector are preferably identical to the second optic filter (18) and the detector (19). There will thus be obtained third and fourth detector output signals (29; 35) which represent the intensity of light emitted from the lamp at a certain point in time.

In both cases as explained above, reference measurements may be carried out either in additional separate measurement cells along separate light beams or, preferably, in the same measurement cells by temporarily replacing the sample medium (40) by the reference medium (40').

The sensitivity range for the concentration measurement may be varied by varying the length of the measurement distance in the sample, i.e. the length of the measurement cell or the measurement space (L). A low concentration requires a longer measurement distance, and vice versa. In order to measure hydrogen peroxide in the gas phase, a longer measurement distance is required, such as from approx. 10 to approx. 250 mm, preferably 50-150 mm and most preferably 25-100 mm. The detection limit on concentration measurement is approx. 0.02 weight % or expressed as 0.2 $g/m^3$ in a gas phase medium.

When the concentration in a gas phase medium is measured linearly, i.e. direct in the gas flow or in the sterilisation chamber in a machine, the longer measurement distances (L) may advantageously be used.

In order to measure hydrogen peroxide at low concentrations, the emission wavelength of the mercury lamp of 254 nm is highly suitable, both in air/gas phase, and in aqueous solution. Well-functioning detectors for this wavelength are photo diodes, adapted to detect at 254 nm.

Concentrations of hydrogen peroxide in the gas phase or water vapour up to approx. 170 mg/l are preferably measured at 254 nm, the length of the measurement distance being from approx. 25 to approx. 100 mm.

Thus, the present invention realises an optimum method and system for producing a gaseous sterilisation medium with improved accuracy and reliability. In addition, there will be realised an automatic and continuous method and system for automatic and continuous regulation of the function of the vaporiser device and thereby of the concentration and quality of the gaseous medium.

By comparing the absorbency of light at two different wavelengths, one preferably in the UV region and the other preferably selected in the visible region of the spectrum (with an Hg lamp suitable at wavelengths longer than 385 nm), disruptive matter, such as dust particles, dirt and aerosols can be detected and compensated for in their disruptive effect. By also measuring the intensity of the light emitted from the light source but which has not yet passed through the measurement sample, simultaneously with absorbency measurements of the light allowed through the sample, at each measured wavelength, the actual concentration may be determined with improved accuracy.

The method and the system according to the present invention are preferably applicable for the sterilisation of packaging materials and different packing or filling machines.

**Claims**

1. A method for the production of a gaseous medium containing a sterilisation agent, which sterilising agent absorbs UV-light but substantially no light from the visible wavelength range, and continuously regulating and monitoring the concentration and quality of the gaseous sterilisation medium, comprising the steps of
vaporising a liquiform medium (101) containing said sterilisation agent;
detecting aerosols and liquid droplets in the gaseous medium before or when the medium reaches a sterilisation space (116), by means of a method selected from light absorption, light scattering and acoustic methods ;

continuously measuring the concentration in the gaseous medium of an agent or substance which absorbs UV light at one or more first wavelength(s) between approx. 220 and approx. 320 nm, in the presence of matter including liquid droplets and/ or aerosols, which absorb light in the UV spectrum and in the visible spectrum,

continuously processing measurement signals from said detecting means and measurement instruments by carrying out calculations and converting them into output signals for continuously regulating and monitoring the function of the vaporiser,

the step of continuously measuring the concentration comprising the further steps of

a) providing a light source (11) which emits light, including light from the UV spectrum as well as from the visible spectrum, and including said first wavelength(s) and at least a second wavelength or wavelength region of approx. 385 nm or longer;

b) directing light from the light source through a sample of a the gaseous medium (40) containing the agent or substance which is to be measured, as well as matter including liquid droplets and/ or aerosols, along a measurement distance of a length (L);

c) measuring the intensity of light (20) allowed through the sample (40) at said first and said second wavelengths, respectively;

d) directing light from the light source through a reference sample of gaseous medium (40'), containing substantially less of the substance or agent which is to be measured, along the a measurement distance of a length (L);

e) measuring the intensity of light (20') allowed through the reference sample (40'), at said first and second wavelength(s) respectively;

f) thus produce first detector output signals (15; 15') in order to indicate the difference in light intensity from sample and reference sample, respectively, at said first wavelength(s) and second detector output signals (22; 22') for correspondingly indicating the difference in light intensity at said second wavelength(s);

g) determining the concentration of the UV-absorbing substance or agent on the basis of the relative values of the first output signals (15, 15') using Beer-Lambert's relationship;

h) correcting the value of the concentration determined in g), on the basis of the second detector output signals (22;22') whereby the effect of matter including liquid droplets and/or aerosols, present in the sample (40) is eliminated,

in which method

i) the intensity of light from said light source which has not passed through said sample medium (40) or reference sample medium (40'), respectively, is detected at said first and said second wavelength(s), respectively, simultaneously with the measurements in c) and e); and

j) said concentration determination in h) is corrected for faults arising out of variations in the intensity of emitted light from the light source taking the measurements in i) as the point of departure.

2. The method as claimed in Claim 1, **characterised in that** it includes the step (115) in which gas of poor quality and incorrect concentration may be removed during occasional periods of time, by means of a bleeder valve (115).

3. The method as claimed in any of Claim 1 or 2, **characterised in that** it moreover includes a step (114) in which aerosols and liquid droplets are removed from the gaseous medium by means of a hot filter (114).

4. The method as claimed in any of Claims 1 to 3, **characterised in that** the aerosols and liquid droplets are detected by light absorption spectrophotometry at the same time as the concentration of the sterilising agent is continuously measured in the one and same step.

5. The method as claimed in any of the preceding Claims, wherein the sterilisation agent is hydrogen peroxide.

6. The method as claimed in any of the preceding Claims, wherein the gaseous medium (40) is based on air and/or water vapour.

7. A system (100) for producing a gaseous medium containing a sterilisation agent, and continuously regulating and monitoring the concentration and quality of the sterilisation medium, comprising:

an apparatus for vaporising (111) a liquiform medium (101) containing said sterilisation agent,

an up-stream a sterilising space (116);

a device for detecting (112; 113) aerosols and liquid droplets, present in the gaseous medium containing a sterilising agent, before or when the gaseous medium reaches the sterilising space (116), by means of a method selected from light absorption, light scattering and acoustic methods;
a device for continuous measurement of the concentration (113) of the sterilising agent in the gaseous medium; and
a calculator unit (117) for processing measurement signals from said detectors and measurement instruments, calculating and converting into output signals for the continuous regulation and monitoring of the function of the vaporiser,
the concentration measurement device (113) comprising an apparatus (10) for continuous concentration determination of a substance or agent which absorbs UV light at one or more first wavelength(s) of between approx. 220 and approx. 320 nm, in a gaseous medium (40) which includes the sterilisation agent which is to be measured, as well as matter including aerosols and/ or droplets, the apparatus including:

a) a light source (11) which emits light including light from the UV spectrum as well as from the visible spectrum, and encompassing said first wavelength(s) and at least a second wavelength or wavelength region of approx. 385 nm or longer;
b) a measurement distance of a length (L) which is to intersects the medium (40);
c) a device for directing the light through the medium (40) along the measurement distance;
d) at least a first detector (14) adapted to measure the intensity of the UV light allowed through along the measurement distance at the first wavelength(s), the first detector generating a first, first detector output signal (15) which represents the intensity of the light allowed through a sample of the gaseous medium (40) containing the agent or substance which is to be measured, as well as matter including aerosols and/ or liquid droplets at said first wavelength(s), and a second, first detector output signal (15') which represents the intensity of the light allowed through a reference sample of the gaseous medium (40') containing none or substantially less of the substance or agent which is to be measured at said first wavelength(s);
e) at least a second detector (19) adapted to measure the intensity of the light allowed through along the measurement distance at said second wavelength(s), the second detector generating a first, second detector output signal (22) which represents the intensity of the light allowed through a sample of the medium (40) containing the substance or agent which is to be measured, as well as matter including aerosols and/or liquid droplets, at said second wavelength(s), and a second, second detector output signal (22') which represents the intensity of the light allowed through a reference sample of the medium (40') containing none or substantially less of the substance or agent which is to be measured, at said second wavelength (s), and
f) a calculator unit (36) for calculating the determined concentration of the UV-absorbing agent on the basis of the relative values of the output signals by employing Beer-Lambert's relationship;
said apparatus (10), for the purpose of compensating for variations in the intensity of the emitted light from said light source, moreover including:
g) at least a third detector (26) designed for measuring the intensity of the UV light before it is transmitted through the sample, at said first wavelength(s), simultaneously with the measurements at the first detector;
h) at least a fourth detector (33) designed for measuring the intensity of the light before it is transmitted through the sample, at said second wavelength(s), simultaneously with the measurements at the second detector; and
i) a calculator unit (36') for correcting said determined concentration for faults caused by variations in the intensity of light emitted from the light source.

8. The system as claimed in Claim 7, **characterised in that** it moreover includes a bleeder valve (115) for removing, under occasional periods of time, gas of poor quality and incorrect concentration.

9. The system as claimed in any one of Claims 7 or 8, **characterised in that** it moreover includes a hot filter (114) for removing aerosols and liquid droplets from in the gaseous medium.

10. The system as claimed in any one of claims 7 to 9, **characterised in that** said device for continuous detecting of aerosols and liquid droplets, by light absorption spectrophotometry, is included in said device (112b) for continuous measurement of the concentration of the sterilising agent in the gaseous medium.

**Patentansprüche**

1. Verfahren zur Herstellung eines gasförmigen Mediums, welches ein Sterilisationsmittel umfasst, das UV-Licht, aber im Wesentlichen kein Licht aus dem sichtbaren Wellenlängenbereich absorbiert, und zum kontinuierlichen Regulieren und Überwachen der Konzentration und Qualität des gasförmigen Sterilisationsmediums, umfassend die folgenden Schritte:

Verdampfen eines Mediums (101) in Flüssigkeitsform, das das Sterilisationsmittel enthält;

Erfassen von Aerosolen und Flüssigkeitstropfen in dem gasförmigen Medium, bevor oder wenn das Medium einen Sterilisationsraum (116) erreicht, mittels eines Verfahrens, das aus Lichtabsorptions-, Lichtstreu- und akustischen Verfahren ausgewählt ist;

kontinuierliches Messen der Konzentration in dem gasförmigen Medium eines Mittels oder einer Substanz, das oder die UV-Licht mit einer oder mehreren ersten Wellenlängen zwischen ungefähr 220 und ungefähr 320 nm absorbiert, in Gegenwart von Stoffen, enthaltend Flüssigkeitstropfen und/oder Aerosole, die Licht im UV-Spektrum und im sichtbaren Spektrum absorbieren,

kontinuierliches Verarbeiten von Messsignalen aus dem Detektionsmittel und den Messgeräten, indem Berechnungen durchgeführt werden und diese in Ausgabesignale umgewandelt werden, zum kontinuierlichen Regulieren und Überwachen der Funktion des Verdampfers,

wobei der Schritt des kontinuierlichen Messens der Konzentration die weiteren folgenden Schritte umfasst:

a) Bereitstellen einer Lichtquelle (11), die Licht abgibt, enthaltend Licht aus dem UV-Spektrum sowie aus dem sichtbaren Spektrum, und enthaltend die erste(n) Wellenlänge(n) und mindestens eine zweite Wellenlänge oder einen zweiten Wellenlängenbereich von ungefähr 385 nm oder mehr;

b) Leiten des Lichts aus der Lichtquelle durch eine Probe des gasförmigen Mediums (40), das das zu messende Mittel oder die zu messende Substanz sowie Stoffe enthaltend Flüssigkeitstropfen und/oder Aerosole enthält, entlang einer Messstrecke einer Länge (L);

c) Messen der Intensität von Licht (20), das mit der oder den ersten bzw. zweiten Wellenlänge(n) durch die Probe (40) gelassen wird;

d) Leiten des Lichts aus der Lichtquelle durch eine Bezugsprobe von gasförmigem Medium (40'), das wesentlich weniger der zu messenden Substanz oder des zu messenden Mittels enthält, entlang der Messstrecke einer Länge (L);

e) Messen der Intensität von Licht (20'), das mit der oder den ersten bzw. zweiten Wellenlänge(n) durch die Bezugsprobe (40') gelassen wird;

f) demgemäßes Erzeugen erster Detektorausgabesignale (15; 15'), um den Unterschied der Lichtintensität der Probe bzw. Bezugsprobe bei der oder den ersten Wellenlänge(n) anzuzeigen und zweiter Detektorausgabesignale (22; 22') zum entsprechenden Anzeigen des Unterschieds der Lichtintensität bei der oder den zweiten Wellenlänge(n);

g) Bestimmen der Konzentration der UV-Licht-absorbierenden Substanz oder des UV-Licht-absorbierenden Mittels auf der Basis der relativen Werte der ersten Ausgabesignale (15, 15') mittels der Lambert-Beerschen Beziehung;

h) Korrigieren des Werts der in Schritt g) bestimmten Konzentration auf der Basis der zweiten Detektorausgabesignale (22; 22'), wodurch der Effekt von in der Probe (40) vorliegenden Stoffen, enthaltend Flüssigkeitstropfen und/oder Aerosole, beseitigt wird, bei welchem Verfahren

i) die Intensität von Licht aus der Lichtquelle, welches nicht durch das Probenmedium (40) bzw. Bezugsprobenmedium (40') getreten ist, bei der oder den ersten bzw. zweiten Wellenlänge(n) gleichzeitig mit den Messungen in Schritt c) und e) erfasst wird; und

j) die Konzentrationsbestimmung in Schritt h) bezüglich Fehler, die aus Schwankungen der Intensität abgegebenen Lichts aus der Lichtquelle stammen, ausgehend von den Messungen in Schritt i) korrigiert wird.

**2.** Verfahren nach Anspruch 1, **dadurch gekennzeichnet, dass** es den Schritt (115) enthält, bei dem Gas schlechter Qualität und inkorrekter Konzentration während gelegentlicher Zeitspannen mittels eines Entlüftungsventils (115) entfernt werden kann.

**3.** Verfahren nach Anspruch 1 oder 2, **dadurch gekennzeichnet, dass** es darüber hinaus einen Schritt (114) enthält, bei dem Aerosole und Flüssigkeitstropfen mittels eines Heißfilters (114) aus dem gasförmigen Medium entfernt werden.

**4.** Verfahren nach einem der Ansprüche 1 bis 3, **dadurch gekennzeichnet, dass** die Aerosole und Flüssigkeitstropfen durch Lichtabsorptionsspektrophotometrie zur gleichen Zeit erfasst werden, wie die Konzentration des Sterilisiermittels kontinuierlich bei dem gleichen Schritt gemessen wird.

**5.** Verfahren nach einem der vorhergehenden Ansprüche, wobei das Sterilisationsmittel Wasserstoffperoxid ist.

6. Verfahren nach einem der vorhergehenden Ansprüche, wobei das gasförmige Medium (40) auf Luft und/oder Wasserdampf basiert.

7. System (100) zum Erzeugen eines gasförmigen Mediums, welches ein Sterilisationsmittel enthält, und zum kontinuierlichen Regulieren und Überwachen der Konzentration und Qualität des Sterilisationsmediums, umfassend:

eine Vorrichtung zum Verdampfen (111) eines Mediums (101) in Flüssigkeitsform, enthaltend das Sterilisationsmittel,
einen stromaufwärts gelegenen Sterilisierraum (116);
eine Einrichtung zum Erfassen (112; 113) von Aerosolen und Flüssigkeitstropfen, die in dem gasförmigen Medium, das Sterilisiermittel enthält, vorliegen, vor oder bei Erreichen des Sterilisierraums (116) mittels eines Verfahrens, das aus Lichtabsorptions-, Lichtstreu- und akustischen Verfahren ausgewählt ist;
eine Einrichtung zum kontinuierlichen Messen der Konzentration (113) des Sterilisiermittels in dem gasförmigen Medium; und
eine Rechnereinheit (117) zum Verarbeiten von Messsignalen aus den Detektoren und den Messgeräten zum Berechnen und Umwandeln in Ausgabesignale für das kontinuierliche Regulieren und Überwachen der Funktion des Verdampfers,
wobei die Konzentrationsmessungseinrichtung (113) eine Vorrichtung (10) zum kontinuierlichen Bestimmen einer Substanz oder eines Mittels, die oder das UV-Licht bei einer oder mehreren ersten Wellenlänge(n) von zwischen ungefähr 220 und ungefähr 320 nm absorbiert, in einem gasförmigen Medium (40) umfasst, das das zu messende Sterilisationsmittel sowie Stoffe, enthaltend Aerosole und/oder Tropfen enthält, wobei die Vorrichtung Folgendes enthält:

a) eine Lichtquelle (11), die Licht abgibt, enthaltend Licht aus dem UV-Spektrum sowie aus dem sichtbaren Spektrum, und umfassend die erste(n) Wellenlänge(n) und mindestens eine zweite Wellenlänge oder Wellenlängenbereich von ungefähr 385 nm oder mehr;
b) Messstrecke einer Länge (L), die das Medium (40) schneiden soll;
c) eine Einrichtung zum Leiten des Lichts durch das Medium (40) entlang der Messstrecke;
d) mindestens einen ersten Detektor (14), der dazu ausgelegt ist, die Intensität des UV-Lichts, das hindurchgelassen wird, bei der oder den ersten Wellenlänge(n) entlang der Messstrecke zu messen, wobei der erste Detektor ein erstes Ausgabesignal (15) des ersten Detektors, das die Intensität des Lichts darstellt, das durch eine Probe des gasförmigen Mediums (40), das das zu messende Mittel oder die zu messende Substanz sowie Stoffe, enthaltend Aerosole und/oder Flüssigkeitstropfen, enthält, bei der oder den ersten Wellenlänge(n) durchgelassen wird, und ein zweites Ausgabesignal (15') des ersten Detektors erzeugt, das die Intensität des Lichts darstellt, das durch eine Referenzprobe des gasförmigen Mediums (40'), das keine oder kein, oder wesentlich weniger, zu messende Substanz oder zu messendes Mittel enthält, bei der oder den ersten Wellenlänge(n) durchgelassen wird;
e) mindestens einen zweiten Detektor (19), der dazu ausgelegt ist, die Intensität des Lichts, das hindurchgelassen wird, bei der oder den zweiten Wellenlänge(n) entlang der Messstrecke zu messen, wobei der zweite Detektor ein erstes Ausgabesignal (22) des zweiten Detektors, das die Intensität des Lichts darstellt, das durch eine Probe des gasförmigen Mediums (40), das die zu messende Substanz oder das zu messende Mittel sowie Stoffe, enthaltend Aerosole und/oder Flüssigkeitstropfen, enthält, bei der oder den zweiten Wellenlänge(n) durchgelassen wird, und ein zweites Ausgabesignal (22') des zweiten Detektors erzeugt, das die Intensität des Lichts darstellt, das durch eine Referenzprobe des gasförmigen Mediums (40'), das keine oder kein, oder wesentlich weniger, zu messende Substanz oder zu messendes Mittel enthält, bei der oder den zweiten Wellenlänge(n) durchgelassen wird, und
f) eine Rechnereinheit (36) zum Berechnen der bestimmten Konzentration des UV-Licht-absorbierenden Mittels auf der Basis der relativen Werte der Ausgabesignale durch Anwenden der Lambert-Beerschen Beziehung;
wobei die Vorrichtung (10), für den Zweck des Kompensierens für Schwankungen der Intensität des abgegebenen Lichts aus der Lichtquelle, des Weiteren Folgendes enthält:
g) mindestens einen dritten Detektor (26), der dazu ausgebildet ist, die Intensität des UV-Lichts zu messen, bevor es durch die Probe tritt, bei der oder den ersten Wellenlänge(n), gleichzeitig mit den Messungen am ersten Detektor;
h) mindestens einen vierten Detektor (33), der dazu ausgebildet ist, die Intensität des UV-Lichts zu messen, bevor es durch die Probe tritt, bei der oder den zweiten Wellenlänge(n), gleichzeitig mit den Messungen am zweiten Detektor; und
i) eine Rechnereinheit (36') zum Korrigieren der bestimmten Konzentration bezüglich Fehlern, die durch Schwankungen der Intensität des aus der Lichtquelle abgegebenen Lichts verursacht werden.

**8.** System nach Anspruch 7, **dadurch gekennzeichnet, dass** es darüber hinaus ein Entlüftungsventil (115) zum Entfernen in gelegentlichen Zeitspannen von Gas schlechter Qualität und inkorrekter Konzentration enthält.

**9.** System nach Anspruch 7 oder 8, **dadurch gekennzeichnet, dass** es darüber hinaus ein Heißfilter (114) zum Entfernen von Aerosolen und Flüssigkeitstropfen aus dem gasförmigen Medium enthält.

**10.** System nach einem der Ansprüche 7 bis 9, **dadurch gekennzeichnet, dass** die Einrichtung zum kontinuierlichen Erfassen von Aerosolen und Flüssigkeitstropfen durch Lichtabsorptionsspektrophotometrie in der Einrichtung (112b) zum kontinuierlichen Messen der Konzentration des Sterilisiermittels in dem gasförmigen Medium enthalten ist.

**Revendications**

**1.** Procédé pour la production d'un milieu gazeux contenant un agent de stérilisation, lequel agent de stérilisation absorbe la lumière UV, mais sensiblement pas la lumière de la gamme des longueurs d'onde visibles, et pour la régulation et le suivi continus de la concentration et de la qualité du milieu de stérilisation gazeux, comprenant les étapes consistant à

vaporiser un milieu sous forme liquide (101) contenant ledit agent de stérilisation ;

détecter les aérosols et les gouttelettes liquides dans le milieu gazeux avant ou pendant que le milieu atteint un espace de stérilisation (116), au moyen d'une méthode choisie parmi l'absorption de lumière, la diffusion de lumière et les méthodes acoustiques ;

mesurer en continu la concentration dans le milieu gazeux d'un agent ou d'une substance qui absorbe la lumière UV à une ou plusieurs premières longueurs d'onde comprises entre environ 220 et environ 320 nm, en présence d'une matière comprenant des gouttelettes liquides et/ou des aérosols qui absorbent la lumière dans le spectre UV et dans le spectre visible ;

traiter en continu les signaux de mesure provenant desdits moyens de détection et instruments de mesure en effectuant des calculs et en les convertissant en signaux de sortie pour réguler et suivre en continu le fonctionnement du vaporiseur,

l'étape de mesure continue de la concentration comprenant les étapes supplémentaires consistant à :

a) obtenir une source lumineuse (11) qui émet une lumière comprenant une lumière du spectre UV ainsi que du spectre visible, et incluant lesdites premières longueurs d'onde et au moins une deuxième longueur d'onde ou région de longueurs d'onde d'environ 385 nm ou plus longues ;

b) faire passer la lumière de la source lumineuse à travers un échantillon de milieu gazeux (40) contenant l'agent ou la substance qui doit être mesuré, ainsi que la matière comprenant des gouttelettes liquides et/ou des aérosols, sur une distance de mesure d'une longueur (L) ;

c) mesurer l'intensité de la lumière (20) ayant traversé l'échantillon (40) auxdites premières et auxdites deuxièmes longueurs d'onde, respectivement ;

d) faire passer la lumière de la source lumineuse à travers un échantillon de référence de milieu gazeux (40') contenant sensiblement moins de la substance ou de l'agent qui doit être mesuré, sur la distance de mesure de longueur (L) ;

e) mesurer l'intensité de la lumière (20') ayant traversé l'échantillon de référence (40') auxdites premières et auxdites deuxièmes longueurs d'onde, respectivement ;

f) générer ainsi des premiers signaux de sortie de détecteur (15 ; 15') afin d'indiquer la différence d'intensité lumineuse entre l'échantillon et l'échantillon de référence, respectivement, auxdites premières longueurs d'onde, et des deuxièmes signaux de sortie de détecteur (22 ; 22') afin d'indiquer proportionnellement la différence d'intensité lumineuse auxdites deuxièmes longueurs d'onde ;

g) déterminer la concentration de la substance ou de l'agent absorbant les UV en fonction des valeurs relatives des premiers signaux de sortie (15 ; 15') en utilisant la loi de Beer-Lambert ;

h) corriger la valeur de la concentration déterminée en g) en fonction des deuxièmes signaux de sortie de détecteur (22 ; 22'), l'effet de la matière comprenant des gouttelettes liquides et/ou des aérosols présente dans l'échantillon (40) étant ainsi supprimé,

dans lequel procédé

i) l'intensité de la lumière de ladite source lumineuse qui n'a pas traversé ledit échantillon de milieu (40) ou ledit échantillon de référence de milieu (40'), respectivement, est détectée auxdites premières et auxdites deuxièmes longueurs d'onde, respectivement, simultanément aux mesures en c) et e) ; et

j) ladite détermination de concentration en h) est corrigée des défauts résultant des variations d'intensité de la lumière émise par la source lumineuse en prenant les mesures en i) au point de départ.

2. Procédé selon la revendication 1, **caractérisé en ce qu'**il comprend une étape (115) dans laquelle le gaz de qualité médiocre et de concentration incorrecte peut être éliminé lors de périodes de temps occasionnelles, au moyen d'un robinet de purge (115).

3. Procédé selon l'une quelconque des revendications 1 et 2, **caractérisé en ce qu'**il comprend en outre une étape (114) dans laquelle les aérosols et les gouttelettes liquides sont éliminés du milieu gazeux au moyen d'un filtre chaud (114).

4. Procédé selon l'une quelconque des revendications 1 à 3, **caractérisé en ce que** les aérosols et les gouttelettes liquides sont détectés par spectrophotométrie d'absorption en même temps que la concentration de l'agent de stérilisation est mesurée en continu dans une seule et même étape.

5. Procédé selon l'une quelconque des revendications précédentes, dans lequel l'agent de stérilisation est le peroxyde d'hydrogène.

6. Procédé selon l'une quelconque des revendications précédentes, dans lequel le milieu gazeux (40) est basé sur l'air et/ou la vapeur d'eau.

7. Système (100) pour produire un milieu gazeux contenant un agent de stérilisation, et réguler et suivre en continu la concentration et la qualité du milieu de stérilisation, comprenant
un appareil (111) pour vaporiser un milieu sous forme liquide (101) contenant ledit agent de stérilisation ;
un espace de stérilisation (116) en amont ;
un dispositif (112 ; 113) pour détecter les aérosols et les gouttelettes liquides présents dans le milieu gazeux contenant l'agent de stérilisation, avant ou pendant que le milieu gazeux atteint l'espace de stérilisation (116), au moyen d'une méthode choisie parmi l'absorption de lumière, la diffusion de lumière et les méthodes acoustiques ;
un dispositif (113) pour mesurer en continu la concentration de l'agent de stérilisation dans le milieu gazeux ; et
une unité de calcul (117) pour traiter les signaux de mesure provenant desdits détecteurs et instruments de mesure, calculer et les convertir en signaux de sortie pour la régulation et le suivi continus du fonctionnement du vaporiseur ;
le dispositif de mesure de concentration (113) comprenant un appareil (10) pour déterminer en continu la concentration d'une substance ou d'un agent qui absorbe la lumière UV à une ou plusieurs premières longueurs d'onde comprises entre environ 220 et environ 320 nm, dans un milieu gazeux (40) qui comprend l'agent de stérilisation qui doit être mesuré, ainsi qu'une matière comprenant des aérosols et/ou des gouttelettes, l'appareil comprenant :

a) une source lumineuse (11) qui émet une lumière comprenant une lumière du spectre UV ainsi que du spectre visible, et incluant lesdites premières longueurs d'onde et au moins une deuxième longueur d'onde ou région de longueurs d'onde d'environ 385 nm ou plus longues ;
b) une distance de mesure d'une longueur (L) qui doit croiser le milieu (40) ;
c) un dispositif pour faire passer la lumière à travers le milieu (40) sur la distance de mesure ;
d) au moins un premier détecteur (14) adapté pour mesurer l'intensité de la lumière UV ayant traversé sur la distance de mesure aux premières longueurs d'onde, le premier détecteur générant un premier signal de sortie du premier détecteur (15) qui représente l'intensité de la lumière ayant traversé un échantillon de milieu gazeux (40) contenant l'agent ou la substance qui doit être mesuré, ainsi que la matière comprenant des aérosols et/ou des gouttelettes liquides auxdites premières longueurs d'onde, et un deuxième signal de sortie du premier détecteur (15') qui représente l'intensité de la lumière ayant traversé un échantillon de référence de milieu gazeux (40') ne contenant pas ou contenant sensiblement moins de la substance ou de l'agent qui doit être mesuré auxdites premières longueurs d'onde ;
e) au moins un deuxième détecteur (19) adapté pour mesurer l'intensité de la lumière ayant traversé sur la distance de mesure auxdites deuxièmes longueurs d'onde, le deuxième détecteur générant un premier signal de sortie du deuxième détecteur (22) qui représente l'intensité de la lumière ayant traversé un échantillon de milieu (40) contenant la substance ou l'agent qui doit être mesuré, ainsi que la matière comprenant des aérosols et/ou des gouttelettes liquides, auxdites deuxièmes longueurs d'onde, et un deuxième signal de sortie du deuxième détecteur (22') qui représente l'intensité de la lumière ayant traversé un échantillon de référence de milieu (40') ne contenant pas ou contenant sensiblement moins de la substance ou de l'agent qui doit être mesuré, auxdites deuxièmes longueurs d'onde ; et
f) une unité de calcul (36) pour calculer la concentration déterminée de l'agent absorbant les UV en fonction des valeurs relatives des signaux de sortie en employant la loi de Beer-Lambert ;

ledit appareil (10), aux fins de compenser les variations d'intensité de la lumière émise par ladite source lumineuse, comprenant en outre :

g) au moins un troisième détecteur (26) conçu pour mesurer l'intensité de la lumière UV avant qu'elle ne soit transmise à travers l'échantillon, auxdites premières longueurs d'onde, simultanément aux mesures au niveau du premier détecteur ;

h) au moins un quatrième détecteur (33) conçu pour mesurer l'intensité de la lumière avant qu'elle ne soit transmise à travers l'échantillon, auxdites deuxièmes longueurs d'onde, simultanément aux mesures au niveau du deuxième détecteur ; et

i) une unité de calcul (36') pour corriger ladite concentration déterminée des défauts résultant des variations d'intensité de la lumière émise par la source lumineuse.

8. Système selon la revendication 7, **caractérisé en ce qu'**il comprend en outre un robinet de purge (115) pour éliminer, lors de périodes de temps occasionnelles, le gaz de qualité médiocre et de concentration incorrecte.

9. Système selon l'une quelconque des revendications 7 et 8, **caractérisé en ce qu'**il comprend en outre un filtre chaud (114) pour éliminer les aérosols et les gouttelettes liquides du milieu gazeux.

10. Système selon l'une quelconque des revendications 7 à 9, **caractérisé en ce que** ledit dispositif pour détecter en continu les aérosols et les gouttelettes liquides, par spectrophotométrie d'absorption, est intégré dans ledit dispositif (112b) pour mesurer en continu la concentration de l'agent de stérilisation dans le milieu gazeux.

Figure 1a

Figure 1b

Figure 2

Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 01244341 A
- US 5872359 A
- US 4296068 A